# EUROPEAN PATENT SPECIFICATION

(11) **EP 0 825 998 B1**
(45) Date of publication and mention of the grant of the patent: **09.04.2003**
(21) Application number: 96913551.6
(22) Date of filing: 07.05.1996
(51) Int. Cl.: C07K 1/36, A61L 2/18, A61K 39/395

(54) **PREPARATION OF IMMUNOGLOBULIN**
HERSTELLUNG VON IMMUNGLOBULIN
PREPARATION DE L'IMMUNOGLOBULINE

(30) Priority: 08.05.1995 FI 952196
(43) Date of publication of application: 04.03.1998
(73) Proprietor: SUOMEN PUNAINEN RISTI VERIPALVELU, 00310 Helsinki (FI)
(72) Inventor: SUOMELA, Hannu, Veli, Herman, FIN-01660 Vantaa (FI); HÄMÄLÄINEN, Eero, Olavi, FIN-00300 Helsinki (FI)
(74) Representative: Heikkinen, Esko Juhani
(86) International application number: FI9600254
(87) International publication number: WO96035710

(56) References cited:
- EP-A- 0 131 740
- EP-A- 0 269 405
- EP-A- 0 378 208
- EP-A- 0 448 075
- US-A- 3 966 906
- US-A- 4 436 724
- US-A- 4 874 708
- VOX SANG., Volume 55, 1988, K.G. REID et al., "Potential Contribution of Mild Pepsin Treatment at pH4 to the Viral Safety of Human Immunoglobulin Products", pages 75-80.
- TRANSFUSION, Volume 31, 1991, C. KEMPF et al., "Virus Inactivation During Production of Intravenous Immunoglobulin", pages 423-427.

## Description

The present invention relates to a method for producing immunoglobulin G. The product can be used in the treatment and prophylaxis of diseases.

The human organism is protected against external pathogenic organisms by an immune system, part of which consists of the immunoglobulins IgG, IgM and IgA. In order to function faultlessly as part of the immune system, the immunoglobulins must be in their native form. Thus the molecular weight of IgG must be about 150 kD, and the so-called Fc part must be unbroken and capable of functioning. IgG can lose its native form in the purification process, for instance as a result from polymerization caused by ethanol. The modified IgG fraction can activate the complement *in vivo* so strongly that the patient for whom the product is infused gets a fatal anaphylactic reaction. The capacity of the IgG fraction to bind the complement is measured *in vitro* as anticomplementary activity.

Immunoglobulin is used in the treatment of certain diseases, for instance idiopathic thrombocytopenia, as well as in protecting from infections patients who lack immunoglobulin G either as a hereditary or temporary feature (hypo- or agamma-globulinemia patients). Specific immunoglobulins, such as products containing antibodies against tetanus, rubella, anti-D or rabies, are used for protecting people against specific diseases.

It has been found out that hepatitis viruses have been transmitted to patients through certain immunoglobulin products. This means that all infective viruses have not been killed in the production process. Hepatitis has been diagnosed in as much as 10 - 20 % of the patients who have received intravenous immunoglobulin treatment.

The best known method for producing intravenous immunoglobulins is treatment with mild pepsin at pH 4 (Barandun et al, Vox Sang 7:157-174, 1962; FI patent 73,597; Suomela et al, Biotechnology of Blood Proteins, 227:261 - 265, 1993.) The aim of the pepsin treatment has been to eliminate anticomplementarity. Other possible methods are for instance intensive fractionating of immunoglobulins by proteolytic enzymes, such as pepsin or plasmin. The protein molecules in the obtained product are so highly fragmented (over half of the molecules) that anticomplementary activity cannot be measured anymore (Römer et al, Develop. Biol. Standard 44:147 - 151, 1979). Protein molecules can also be treated by chemical agents, for example by sulfonating, reducing and alkylating or precipitating with polyethylene glycol or its derivatives (Römer et al., Vox Sang 42:62 - 73, 1982).

Hepatitis viruses can be inactivated by a solvent-detergent (S/D) treatment (US patents 4,591,505, 4,613,501, 4,481,189; Horowitz et al, Int. Assoc. Biol. Standard, 1992 Nov. 9).

In general, patients tolerate immunoglobulin treated with mild pepsin fairly well, but hepatitis transmissions from products made according to this method have been documented (Williams et al, Vox Sang 57:15 - 18, 1989).

Among the drawbacks of particularly enzymatically fractionated or chemically treated products, let us mention shortened biological half-life and inefficiency (Römer et al, Vox Sang 42:62- 73, 1982).

US 4,874,708 describes purification of an immunoglobulin fraction by polyethylene glycol (PEG). PEG is a precipitation agent. Ultrafiltration is used to concentrate the solution of gamma-globulins and diafiltration is used to remove the PEG.

EP-A-448075 relates to a process for producing immunoglobulin G preparations. A porous polyolefin membrane filter with a pore size preferably within 0.015 to 0.17 µm is used in order to remove anticomplementary aggregates.

### DESCRIPTION OF THE INVENTION

The present invention introduces a method for producing immunoglobulin G according to claim 1 below. A few advantageous modifications of the invention are enlisted in the additional claims.

In order to eliminate anticomplementarity and inactivate viruses, the method comprises successive pepsin and S/D treatments, whereafter the chemicals and pepsin used in the inactivation, and the decomposition products of immunoglobulin, are removed. Moreover, in order to eliminate viruses, there is performed filtering with a perforated filter, where the maximum perforation size is no larger than 35 nm.

The pepsin treatment is such that it causes slight proteolysis or distinctive digestion, and advantageously of a kind used in the manufacture of products meant for clinical use. The treatment is performed at pH 3.8 - 4.6 for a suitable duration in conditions where the pepsin is enzymatically active. Most advantageously the treatment period is exceptionally long, about 60 - 72 hours with mild pepsin at the temperature of about 33-40 °C, and the treatment is carried out at pH 4.2 - 4.5, which is higher than normal. A long treatment period has been found out to increase the virus inactivation. A long treatment period also increases the quantity of decomposition products. This is not, however, harmful in the end product, because the decomposition products are removed.

Highly splitted products (for example ad 70 %) can also be manufactured with this method. In the solvent-detergent treatment there is used a solvent-detergent combination that decomposes the lipid envelope of viruses. There is normally used 0.3 % tri(n-butyl)phosphate and 1 % polysorbate (for example Tween 80), 1 % octoxynole (for example Triton 100) or 0.2 % sodium cholate, or 2 % tri(n-butyl)phosphate only. The treatment period is normally 4 - 6 hours, and the temperature 24 - 37 °C.

The chemicals, pepsin and split products are best removed simultaneously by binding the immunoglobulin to an ion exchanger, most advantageously cation exchanger. An agarose type of cation exchanger (for example CM-Sepharose) is most advantageously used.

It was found out that virus filtering effectively eliminates such viruses that are not inactivated in the S/D treatment. Filtering can be done at any stage whatever. Most advantageously it is carried out after removing the chemicals and decomposition products, or during pepsin incubation. Surprisingly it was also discovered out that pepsin treatment remarkably enhances the penetration of proteins in the filtering.

The size of the perforations in the filters used in virus removal (for example nanofilter Planova, Asahi Chemical Co., Japan) is 35 nm at maximum, for instance no larger than 20 nm, most advantageously no larger than 15 nm.

The suggested method combination effectively also eliminates non-enveloped viruses and small enveloped viruses.

Most advantageously the product is finally stabilized, for instance by adding mono- or disaccharide or sugar alcohol.

The original material can be blood plasma fractions manufactured by some known method and containing immunoglobulin G, for instance fractions purified with polyethylene glycol or chromatography. It can be normal or hyperimmune plasma, or a fraction purified from placenta. In particular, the original material can be a Cohn fraction II, which is most advantageously further purified with anion exchanger and freeze-dried from ethanol. It is also possible to use a powder or a solution of immunoglobulin G produced by some other method, for instance a supernatant III of the Cohn fraction or a Cohn fraction II which is not further treated, with electrophoretic purity of over 90 %. The ethanol in ethanol-containing fractions can be removed for instance by ultrafiltering, gel filtering or freeze-drying prior to the dissolution of the immunoglobulin.

Anion exchange treatment (for instance DEAE-Sephadex) is advantageously used as one step in the purification of the Cohn fraction.

By means of the invention, there are obtained extremely pure and well tolerated intravenously administered products that do not contain pepsin and have a lower anticomplementary activity than any of the prior art products. At least 95 %, advantageously at least 98 % of the proteins in the product are IgG, and at least 90 % of the IgG is monomer or dimer. The quantity of polymeric IgG is less than 1 %, and the quantity of small fragments is less than 5 %. According to the invention, it is possible to obtain products with an IgA content below 5 mg/l, typically 2 - 3 mg/l. The prekallikrein activator content is typically less than 1 IU/ml. In addition, the viral safety of the products is secured in more ways than in the prior art products.

The method can be applied to the production of both normal and specific immunoglobulins.

For a liquid preparation, there is extracted some solution after the cation exchange chromatography, and this solution is, for instance by means of pH adjustment, concentration, washing and filtering, manufactured to a product to be preserved as a liquid preparation.

The product may be concentrated and constant-volume washed in an ultrafiltering device with a filter that is permeable to proteins smaller than about 150 kD.

The method according to the invention is realized for instance as follows:

Immunoglobulin is dissolved to an aqueous solution. The pH is adjusted with a mild acid to be about 4.4. Most advantageously the acid is added to the solution in particles as fine as possible. Thus the pH is adjusted rapidly, without damaging the immunoglobulin. Pepsin is added at a weight ratio of about 1:10,000. The solution is incubated about 66 hours at a temperature of roughly 35 °C.
There is added a mixture of the solution and the detergent, and the incubation is continued for at least 8 hours at a temperature of 26 ± 2 °C.
Immunoglobulin is bound to the cation exchanger and eluated with a biologically compatible buffer.
The pH of the eluate is adjusted at about 6.9. The eluate is concentrated, constant-volume washed and at the same time equilibrated to contain 3 - 15 %, advantageously 8 - 9 % saccharose, clarification filtered, portioned out and freeze-dried.
The obtained result is a dry goods product which is turned into an injection solution by adding water.

The injection solution manufactured according to the above description was given to 15 patients in 7 different hospitals. It was found out that the product caused less side effects than the two reference products in current use. The participants in this trial were patients who had earlier received treatment with a reference product for hypogammaglobulinemia. There was not detected any transmission of infective viruses as a result of using the product.

The immunoglobulin solution preserved as a liquid preparation is manufactured for instance as follows:
The original material is the solution obtained from the above described method, prior to freeze-drying, most advantageously after cation exchange. The pH of the eluate obtained from the column is adjusted and the solution is concentrated by ultrafiltering. The chosen ultrafilter is advantageously of a type that is permeable to proteins with a molecular size smaller than about 150 kD. The solution is concentrated to protein content 2 - 10 % (w/w), most advantageously about 5 - 6 %. When necessary, there are added filtering agents, for instance Al(OH)₃ gel and/or diatomaceous earth, and mixed. The filtering agents are removed by filtering or centrifugation.
The solution is clarification filtered and thereafter filtered in a filter with a perforation size of 15 nm.
After filtering, 10 % (w/w) saccharose is dissolved in the solution, the pH is checked and, when necessary, adjusted. Thereafter it is sterile filtered and bottled. The solution should be kept at the temperature of 2 - 8 °C.

When manufacturing a product to be preserved as liquid preparation, it is possible to avoid the freeze-drying step, which sets limits to production and increases expenses.

### EXAMPLE 1

A Cohn fraction II of the material is purified with a DEAE-Sephadex anion exchanger in the following conditions: the pH of gel and mild acetate buffer is 6.85 ± 0.05, temperature 6 - 8 °C, processing period 3 hours. For a kilo of material, there is used 35 g dry anion exchanger.
2.66 kg purified and freeze-dried Cohn fraction II powder is dissolved to 35.64 liters of 10 % saccharose solution containing 0.2 M NaCl. It is cleared by filtering. The pH of the solution is adjusted with 0.2 M HCl at 0 °C to 4.4, and there is added 240 mg purified porcine pepsin. The solution is cleared by filtering. The solution is incubated for 66 hours at a temperature of 35 °C. The pH is adjusted to 5.0 with 0.2 M NaOH at 0 °C and sterile filtered.
There then is added 461 g Tween 80 and 135 g tri(n-butyl)phosphate to the solution, and it is mixed at 26 °C for one hour.
The solution is transported by a peristaltic pump to a virus-free production area, where it is further kept in a closed container for at least 6 - 20 hours. In this area, the production uses only autoclaved equipment or equipment purified of viruses in some other manner. The solution charges a 40 1 CM-Sepharose-FF column, which is equilibrated with a 50 mM acetate buffer, pH 5.0. Tween 80 and the tri(n-butyl)phosphate, pepsin and part of the immunoglobulin decomposition products flow through. The immunoglobulin attached to the column is eluated with a 15 mM sodium acetate buffer, pH 5.0, containing 0.5 M NaCl.
The pH of the solution is adjusted to 6.9 with 0.2 M NaOH, and the solution is concentrated to about 40 liters by ultrafiltering. The protein content and salt composition of the solution is changed by constant-volume washing with 180 liters of a solution containing 8 % (w/v) saccharose, 0.8 % glysin and 60 mM NaCl. There is performed clarification filtering, and filtering with a 15 nm filter. After sterile filtering, the solution is either freeze-dried into the final bottle and closed in a vacuum, or manufactured into a product preserved as a liquid preparation.

### EXAMPLE 2

A Cohn fraction III of supernatant or non-freeze-dried Cohn fraction II solution is ultrafiltered or gel filtered in order to eliminate ethanol and treated with a DEAE ion exchanger in order to remove impurities. The solution is equilibrated by constant-volume washing to a solution with 5 - 10 % (w/v) saccharose and 0.2 % (w/v) NaCl. Production is continued according to the method described in example 1.

### EXAMPLE 3

The pH of the CM-Sepharose eluate produced according to example 1 or 2 is adjusted to 5.1 with 0.2 M NaOH at 0 °C. The solution is concentrated in relation to protein to 5 - 10 % by ultrafiltering with a membrane filter that permeates all molecules smaller than 150 kD. There is performed constant-volume washing with 5 - 8 volumes distilled water.
When desired, there are added, as filtering agents, Al(OH)₃ gel 10 - 30 ml/l and diatomaceous earth filtering agent (for instance Filtercel) 5 - 40 g/l. The filtering agents are removed by centrifugation or advantageously by filtering in connection with the clarification filtering.

The solution is clarification filtered first with a preliminary filter made of glass fiber, and then with 220 nm and 100 nm membrane filters. Next it is filtered in a 15 nm filter. To the solution there is added 10 % (w/v) saccharose, after the dissolution of which the pH is checked and when necessary, the pH is adjusted to 1.5 with NaOH or HCl. The solution is sterile filtered and bottled.

### REFERENCE EXAMPLE

Of a production-scale batch (4 kg immunoglobulin) there was extracted 300 ml solution from the production step prior to the pepsin digestion. The solution was divided into two parts, A and B. Part A was subjected to pepsin digestion in conditions where the pepsin ratio was 1/10,000 of the immunoglobulin weight, pH 4.4, temperature 37 °C, duration 66 hours. Part B was treated in the same manner, but without pepsin. After incubation, the pH of both solutions was adjusted to 5.0. Both parts were filtered successively with the same equipment and filter. The transmembrane pressure was adjusted to 0.5 bar. In the filtering of part A, there was first performed a 30 min stabilizing filtration with a filtering solution. When the filtering speed was stabilized, filtering was continued for 90 minutes. The average filtering rate (mean protein flux) for solution A was 168.4 gh⁻¹m⁻². The equipment was carefully washed with solution B, and there was carried out a 30 min stabilization filtration and a 90 min filtration. The mean protein flux was 58.5 gh⁻¹m⁻².
Thus the filtered protein quantity of pepsin digerated immunoglobulin per time unit was threefold as compared to an undigerated product.

## Claims

1. A method for producing an immunoglobulin G preparation from an immunoglobulin G fraction isolated from blood, **characterized in that**
- in order to eliminate anticomplementarity and inactivate viruses, the fraction is treated with pepsin at pH 3.8- 4.6,
- whereafter the fraction is treated with the solvent/detergent virus inactivation method in order to inactivate viruses, in which method the immunoglobulin G fraction is treated with tri(n-butyl)phosphate and polysorbate, octoxynole, or tri(n-butyl)phosphate only, which decomposes the lipid envelope of viruses,
- whereafter the chemicals and pepsin used in the virus inactivation, and immunoglobulin decomposition products are removed by treating with an ion exchanger, and that
- in addition to this, at some stage after the pepsin treatment, most advantageously also after the removal of the chemicals, pepsin and immunoglobulin decomposition products used for virus inactivation, the fraction is filtered through a perforated filter with perforations no larger than 35 nm, for instance no larger than 20 nm, most advantageously no larger than 15 nm.

2. A method according to claim 1, **characterized in that** the pepsin treatment constitutes treatment for about 60 - 72 hours with pepsin at the temperature of 33 - 40 °C, at pH 4.2 - 4.5, most advantageously at pH 4.4.

3. A method according to claim 1 or 2, **characterized in that** the chemicals, pepsin and immunoglobulin decomposition products used in virus inactivation are removed by treating with a cation exchanger.

4. A method according to claim 3, **characterized in that** the immunoglobulin is attached to the cation exchanger and eluated from it by a buffer with a pH of 5.

5. A method according to any of the claims 1 - 4, **characterized in that** the product also is concentrated, preferably by ultrafiltering, to a content of 2 - 10 w/w %, most advantageously to 5 - 6 w/w %, and sterile filtered.

6. A method according to claim 5, **characterized in that** the product is concentrated and constant-volume washed in an ultrafiltering device with a filter that is permeable to proteins smaller than about 150 kD.

7. A method according to claim 2 and 4 or 5, **characterized in that** the filtering with a perforated filter of 35 nm at maximum is carried out after concentration, prior to sterile filtering.

8. A method according to any of the claims 1 - 7, **characterized in that** the product also is freeze-dried to a product to be preserved dry, or that the product also is processed to a product to be preserved as a liquid preparation.

9. A method according to claim 8, **characterized in that** the product also is processed to a product to be preserved as a liquid preparation and has an IgA content under 5 mg/l, for instance 2 - 3 mg/l.

10. A method according to any of the claims 1 - 9, **characterized in that** the original material is a Cohn fraction purified with an anion exchanger.

## Patentansprüche

1. Verfahren zur Herstellung einer Immunglobulin-G-Zubereitung aus einer aus Blut isolierten Immunglobulin-G-Fraktion, **dadurch gekennzeichnet, dass**
- die Fraktion bei pH 3.8-4.6 mit Pepsin behandelt wird, um Anti-Komplement-Aktivität zu eliminieren und Viren zu inaktivieren,
- danach die Fraktion mit dem Solvens/Detergens-Virus-Inaktivierungsverfahren behandelt wird, um Viren zu inaktivieren, in welchem Verfahren die Immunglobulin-G-Fraktion mit Tri(n-butyl)phosphat und Polysorbat, Octoxynol, oder mit Tri(n-butyl)phosphat alleine behandelt wird, was die Lipidhülle von Viren abbaut,
- danach die Chemikalien und das Pepsin, welche für die Virus-Inaktivierung verwendet wurden, und Immunglobulinabbauprodukte durch Behandlung mit einem Ionenaustauscher entfernt werden, und dass
- zusätzlich dazu in einem Arbeitsgang nach der Pepsinbehandlung, am vorteilhaftesten auch nach der Entfernung der für die Virus-Inaktivierung verwendeten Chemikalien, des Pepsins und der Immunglobulinabbauprodukte, die Fraktion durch einen perforierten Filter mit Perforierungen, die nicht größer als 35 nm sind, z.B. nicht größer als 20 nm, am vorteilhaftesten nicht größer als 15 nm, filtriert wird.

2. Verfahren nach Anspruch 1, **dadurch gekennzeichnet, dass** die Pepsinbehandlung eine etwa 60-72 Stunden lange Behandlung mit Pepsin bei einer Temperatur von 33-40°C bei pH 4.2-4.5, am vorteilhaftesten bei pH 4.4, darstellt.

3. Verfahren nach Anspruch 1 oder 2, **dadurch gekennzeichnet, dass** die für die Virus-Inaktivierung verwendeten Chemikalien, das Pepsin und die Immunglobulinabbauprodukte durch Behandlung mit einem Kationenaustauscher entfernt werden.

4. Verfahren nach Anspruch 3, **dadurch gekennzeichnet, dass** das Immunglobulin an den Kationenaustauscher bindet und davon mit einem Puffer mit einem pH von 5 eluiert wird.

5. Verfahren nach einem der Ansprüche 1-4, **dadurch gekennzeichnet, dass** das Produkt auch konzentriert wird, vorzugsweise durch Ultrafiltration, bis zu einem Gehalt von 2-10 Gew.-%, am vorteilhaftesten bis zu 5-6 Gew.-%, und sterilfiltriert wird.

6. Verfahren nach Anspruch 5, **dadurch gekennzeichnet, dass** das Produkt konzentriert wird und in einer Ultrafiltrationsvorrichtung mit einem Filter, der für Proteine kleiner als etwa 150 kD permeabel ist, bei konstantem Volumen gewaschen wird (is constant-volume washed).

7. Verfahren nach Anspruch 2 und 4 oder 5, **dadurch gekennzeichnet, dass** die Filtration mit einem perforierten Filter von max. 35 nm nach der Konzentration, vor der Sterilfiltration, durchgeführt wird.

8. Verfahren nach einem der Ansprüche 1-7, **dadurch gekennzeichnet, dass** das Produkt auch zu einem Produkt, das trocken konserviert werden soll, gefriergetrocknet wird, oder dass das Produkt auch zu einem Produkt, das als flüssige Zubereitung konserviert werden soll, prozessiert wird.

9. Verfahren nach Anspruch 8, **dadurch gekennzeichnet, dass** das Produkt auch zu einem Produkt, das als flüssige Zubereitung konserviert werden soll, prozessiert wird und einen IgA-Gehalt unter 5 mg/l hat, z.B. 2-3 mg/l.

10. Verfahren nach einem der Ansprüche 1-9, **dadurch gekennzeichnet, dass** das Ausgangsmaterial eine Cohn-Fraktion ist, die mit einem Anionenaustauscher aufgereinigt wurde.

## Revendications

1. Méthode de production d'une préparation d'immunoglobuline G à partir d'une fraction d'immunoglobuline G isolée du sang, **caractérisée en ce que** :
- afin d'éliminer l'anticomplémentarité et d'inactiver les virus, la fraction est traitée avec de la pepsine à pH 3,8 - 4,6,
- après quoi la fraction est traitée avec la méthode d'inactivation des virus au solvant/détergent afin d'inactiver les virus, méthode suivant laquelle la fraction d'immunoglobuline G est traitée avec du tri(n-butyl)phosphate et du polysorbate, de l'octoxynole, ou du tri(n-butyl)phosphate seul, ce qui a pour effet de décomposer l'enveloppe lipidique des virus,
- après quoi les agents chimiques et la pepsine utilisés pour l'inactivation des virus, et les produits de décomposition de l'immunoglobuline sont éliminés par traitement avec un échangeur d'ions,
- et en addition à cela, dans une étape postérieure au traitement à la pepsine, le plus avantageusement également postérieure à l'élimination des agents chimiques, de la pepsine et des produits de décomposition de l'immunoglobuline utilisés pour l'inactivation des virus, la fraction est filtrée au travers d'un filtre perforé de perforations de taille inférieure à 35 nm, par exemple inférieure à 20 nm, et plus avantageusement inférieure à 15 nm.

2. Méthode selon la revendication 1, **caractérisée en ce que** le traitement à la pepsine consiste en un traitement avec de la pepsine durant 60-72 heures, à une température de 33-40 °C, à un pH de 4,2-4,5, plus avantageusement à pH 4,4.

3. Méthode selon la revendication 1 ou 2, **caractérisée en ce que** les agents chimiques, la pepsine et les produits de décomposition de l'immunoglobuline utilisés pour l'inactivation des virus sont éliminés par traitement avec un échangeur de cations.

4. Méthode selon la revendication 3, **caractérisée en ce que** l'immunoglobuline est attachée à l'échangeur de cations et en est éluée au moyen d'un tampon de pH 5.

5. Méthode selon l'une quelconque des revendications 1 à 4, **caractérisée en ce que** le produit est également concentré, de préférence par ultrafiltration, jusqu'à une teneur de 2-10 % en poids par rapport au poids total de la préparation, plus avantageusement de 5-6 % en poids par rapport au poids total de la préparation, et stérilisé par filtration.

6. Méthode selon la revendication 5, **caractérisée en ce que** le produit est concentré et lavé à volume constant dans un dispositif d'ultrafiltration avec un filtre qui est perméable aux protéines de taille inférieure à environ 150 kD.

7. Méthode selon la revendication 2 et 4 ou 5, **caractérisée en ce que** la filtration avec un filtre perforé de perforations de 35 nm au maximum est réalisée après la concentration, avant la filtration stérile.

8. Méthode selon l'une quelconque des revendications 1 à 7, **caractérisée en ce que** le produit est aussi lyophilisé pour donner un produit devant être conservé sec, ou **en ce que** le produit est aussi transformé en un produit devant être conservé en préparation liquide.

9. Méthode selon la revendication 8, **caractérisée en ce que** le produit est aussi transformé en un produit devant être conservé en préparation liquide et **en ce qu'**il a une teneur en IgA inférieure à 5 mg/l, par exemple comprise entre 2 et 3 mg/l.

10. Méthode selon l'une quelconque des revendications 1 à 9, **caractérisée en ce que** le matériel d'origine est une fraction de Cohn purifiée avec un écharigeur d'anions.
